# EUROPÄISCHE PATENTANMELDUNG

(11) **EP 4 613 727 A1**
(43) Veröffentlichungstag der Anmeldung: **10.09.2025**
(21) Anmeldenummer: 24161214.2
(22) Anmeldetag: 04.03.2024
(51) Int. Cl.: C07C 2/08, C07C 11/02, C07C 7/04

(54) **ENERGIEEFFIZIENTES VERFAHREN ZUR OLIGOMERISIERUNG VON OLEFINEN**

(71) Anmelder: Evonik Oxeno GmbH & Co. KG, 45772 Marl (DE)
(72) Erfinder: PAUL, Niklas, 45770 Marl (DE); RIX, Armin, Matthias, 45770 Marl (DE); SIX, Tanita Valèrie, 44309 Dortmund (DE); STEENWEG, Tanja, 44139 Dortmund (DE); PEITZ, Stephan, 45739 Oer-Erkenschwick (DE); STOCHNIOL, Guido, 45721 Haltern am See (DE)
(74) Vertreter: Evonik Patent Association

(57) **Zusammenfassung**

Die vorliegende Erfindung betrifft ein Verfahren zur Oligomerisierung von C2- bis C8-Olefinen in mindestens zwei Reaktionsstufen, die jeweils mindestens einen Reaktor und mindestens eine Destillationskolonne umfassen, und eine sich anschließende Abtrennstufe, die mindestens eine Destillationskolonne umfasst, wobei Reaktionswärme und/oder die Kondensationsenergie mithilfe eines Wärmeträgers nutzbar gemacht wird.

## Beschreibung

Gegenstand der Erfindung ist ein Verfahren zur Oligomerisierung von C2- bis C8-Olefinen in mindestens zwei Reaktionsstufen, die jeweils mindestens einen Reaktor und mindestens eine Destillationskolonne umfassen, und eine sich anschließende Abtrennstufe, die mindestens eine Destillationskolonne umfasst, wobei Reaktionswärme und/oder die Kondensationsenergie mithilfe eines Wärmeträgers nutzbar gemacht wird.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen (Propen) ein Olefin mit sechs Kohlenstoffatomen (Hexen) aufbauen. Die Oligomerisierung von zwei Molekülen miteinander wird auch Dimerisierung genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten Katalysator, heterogen an einem festen Katalysator oder mit einem zweiphasigen Katalysatorsystem durchgeführt.

Verfahren zur Oligomerisierung von Olefinen sind im Stand der Technik hinlänglich bekannt und werden großindustriell eingesetzt. Die Produktionsmengen belaufen sich allein in Deutschland auf mehrere tausend Kilotonnen pro Jahr. Um möglichst hohe Umsätze und einen möglichst kontinuierlichen Betrieb von Oligomerisierungsverfahren zu ermöglichen, weisen industrielle Anlagen meist nicht nur eine, sondern mehrere hintereinandergeschaltete Reaktionsstufen auf, die jeweils mindestens einen Reaktor umfassen. Dadurch kann das Oligomerisierungsverfahren selbst bei Ausfall einer Reaktionsstufe weiterbetrieben werden.

Eine Reaktionsstufe umfasst weiterhin mindestens eine Destillationskolonne, um die gebildeten Oligomere von den eingesetzten Olefinen abzutrennen. Dabei wird der Einsatzolefin-haltige Strom als Destillat über Kopf genommen und über den Sumpf das an Einsatzolefinen abgereicherte Oligomerisat abgeführt. Bei der Abtrennung der entstandenen Oligomere nach der Reaktion müssen in industriellen Anlagen hohe Mengen an Energie, beispielsweise in Form von Heizdampf zur Beheizung der Destillationskolonnen, zugeführt werden. Abgesehen von den hohen Kosten für die Bereitstellung des Heizdampfes hat diese Verfahrensführung hohe CO₂-Emissionen zur Folge. Darüber hinaus kann es auch Probleme geben, wenn der Umsatz der Reaktion erhöht werden soll. Zur Umsatzerhöhung und nachhaltigen Ausnutzung des Feedstroms kann der von den Oligomeren abgereicherte Strom, der am Kopf der Destillationskolonne entnommen wird, zumindest teilweise zur ersten Reaktionsstufe zurückgeführt werden. Jedoch muss dieser Strom nach der Umsetzung wieder in der Kolonne prozessiert werden, was zu einem zusätzlichen Einsatz von Energie in Form von Heizdampf führt.

Die Aufgabe der vorliegenden Erfindung war die Bereitstellung eines Verfahrens zur Oligomerisierung von Olefinen, welches die vorgenannten Probleme nicht aufweist. Das Verfahren sollte kosten- und CO₂-sparender betrieben werden können und auch ressourcenschonender bei möglichen Maßnahmen zur Umsatzerhöhung betrieben werden können.

Die Aufgabe konnte durch das erfindungsgemäße Verfahren zur Oligomerisierung von C2- bis C8-Olefinen gemäß Anspruch 1 gelöst werden. Bevorzugte Ausgestaltungen sind in den Unteransprüchen angegeben.

Das erfindungsgemäße Verfahren ist ein Verfahren zur Oligomerisierung von C2- bis C8-Olefinen in mindestens zwei Reaktionsstufen R1 und RL, die jeweils mindestens einen Reaktor und mindestens eine Destillationskolonne umfassen, und einer sich an RL anschließenden Abtrennstufe A1, die mindestens eine Destillationskolonne DKA1 umfasst, wobei
ein Einsatzgemisch, welches zumindest die C2- bis C8-Olefine als Eduktolefine enthält, in dem mindestens einen Reaktor der ersten Reaktionsstufe R1 unter Verwendung eines heterogenen Katalysators einer Oligomerisierung unterworfen wird und das aus dem mindestens einen Reaktor erhaltene Reaktionsgemisch in der mindestens einen Destillationskolonne DKR1 der ersten Reaktionsstufe R1 destilliert wird, wodurch ein Reststrom RS1, der zumindest nicht umgesetzte Eduktolefine enthält, und ein Oligomerisat O1, das zumindest die gebildeten Oligomere enthält, anfallen,
RS1 zumindest teilweise zu einem Reaktor einer nachfolgenden Reaktionsstufe geführt wird;
ein Reststrom aus einer vorherigen Reaktionsstufe in dem mindestens einen Reaktor der letzten Reaktionsstufe RL unter Verwendung eines heterogenen Katalysators einer Oligomerisierung unterworfen wird und das aus dem mindestens einen Reaktor erhaltene Reaktionsgemisch in der mindestens einen Destillationskolonne DKRL der letzten Reaktionsstufe RL destilliert wird, wodurch ein Reststrom RSL, der zumindest nicht umgesetzte Eduktolefine enthält, und ein Oligomerisat OL, das zumindest die gebildeten Oligomere enthält, anfallen;
OL wird zumindest teilweise zur Destillationskolonne DKA1 der sich anschließenden Abtrennstufe A1 geleitet und dort destilliert, wodurch ein Dimerstrom D1, der zu mindestens 90 Gew.-% aus Dimeren der eingesetzten Eduktolefine besteht, und ein Sumpfstrom S1, der zumindest Trimere und höhere Oligomere der eingesetzten Eduktolefine enthält, anfallen;
der Dimerstrom D1 am Kopf der DKA1 anfällt, kondensiert wird und anschließend teilweise als Rücklauf zur DKA1 zurückgeführt und teilweise aus dem Verfahren ausgeschleust wird,
die bei der Kondensation des Dimerstroms D1 entstehende Energie und die im mindestens einen Reaktor der ersten Reaktionsstufe R1 entstehende Wärmeenergie zumindest teilweise auf einen flüssigen oder gasförmigen Wärmeträger W1 übertragen werden, wodurch ein erwärmter Wärmeträger W1.1 entsteht; und
Energie vom erwärmten Wärmeträger W1.1 auf einen weiteren flüssigen oder gasförmigen Wärmeträger W2 übertragen wird, wodurch ein erwärmter Wärmeträger W2.1 entsteht, der zunächst verdichtet und/oder erwärmt wird und über den anschließend mithilfe einer ein- oder mehrstufigen Verdichtung ein Dampfstrom erzeugt wird, mit dem zumindest die Destillationskolonnen DKA1 und DKR1 beheizt werden.

Der Begriff "Reaktionsstufe" meint im Sinne der vorliegenden Erfindung einen Anlagenabschnitt, der jeweils mindestens einen Reaktor und mindestens eine Destillationskolonne umfasst. Eine Reaktionsstufe kann also einen oder mehrere Reaktor(en) und eine oder mehrere auf den oder die Reaktor(en) nachfolgende Destillationskolonne(n) umfassen. In einer bevorzugten Ausführungsform ist nur eine Destillationskolonne pro Reaktionsstufe vorhanden, die auf den oder die Reaktoren folgt. Es ist weiterhin bevorzugt, dass in einer Reaktionsstufe mindestens 2 Reaktoren, besonders bevorzugt exakt 2 Reaktoren angeordnet sind. Die Reaktoren können dabei parallel oder in Reihe geschaltet vorliegen.

In den Destillationskolonnen der jeweiligen Reaktionsstufen werden die erzeugten Oligomere von dem restlichen Ausgangsstrom aus dem Reaktor, welcher beispielsweise Alkane und nicht umgesetzte Olefine umfasst, getrennt. Die Oligomere sind höhersiedend als die nicht umgesetzten Olefine und weitere im Ausgangsstrom aus dem Reaktor befindliche Stoffe und werden deshalb im Sumpf der Destillationskolonne angereichert, während die leichter siedenden nicht umgesetzten Olefine und ggf. im Einsatzstrom vorhandene analoge Alkane über Kopf der Destillationskolonne gehen und im Destillat angereichert werden.

Der Vorteil des erfindungsgemäßen Verfahrens ist, dass durch den Einsatz der Wärmeträger gemäß Anspruch 1 oder gemäß den bevorzugten Ausführungsformen erhebliche Mengen an extern zuzuführender Energie, hauptsächlich in Form von Heizdampf, eingespart werden können. Dadurch kann das Verfahren kostengünstiger und COz-sparender betrieben werden.

Das Einsatzgemisch für das erfindungsgemäße Verfahren enthält zumindest die C2- bis C8-Olefine, bevorzugt C3- bis C6-Olefine, weiterhin bevorzugt C3- bis C5-Olefine, besonders bevorzugt C4-Olefine und deren analoge Alkane. Geeignete Olefine sind unter anderem α-Olefine, n-Olefine und Cycloalkene, vorzugsweise n-Olefine. In einer bevorzugten Ausführungsform handelt es sich bei dem Olefin um n-Buten.

Die Olefine werden üblicherweise nicht in reiner Form als Edukte eingesetzt, sondern in technisch verfügbaren Mischungen. Der in dieser Erfindung zusätzlich verwendete Begriff Einsatzgemisch ist deshalb so zu verstehen, dass er jegliche Art von Gemischen betrifft, die die entsprechenden zu oligomerisierenden Olefine in einer Menge enthalten, die es ermöglicht, die Oligomerisierung wirtschaftlich durchzuführen. Bevorzugt enthalten die erfindungsgemäß verwendeten Einsatzgemische praktisch keine weiteren ungesättigten Verbindungen und mehrfach ungesättigte Verbindungen wie Diene oder Acetylenderivate. Vorzugsweise werden Einsatzgemische eingesetzt, die weniger als 5 Gew.-%, insbesondere weniger als 2 Gew.-% an verzweigten Olefinen bezogen auf den Olefinanteil enthalten.

Propylen wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem C₄-Strom entfernt, z. B. durch Herstellung von MTBE durch Umsetzung mit Methanol. Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Einsatzgemisch zugeführt. Geeignete C4-Olefingemische sind insbesondere das Raffinat I, das Raffinat II und das Raffinat III.

Mit C4-Olefingemischen als Einsatzstrom ergibt sich das folgende besonders bevorzugte Verfahren: Verfahren zur Oligomerisierung von Butenen in mindestens zwei Reaktionsstufen R1 und RL, die jeweils mindestens einen Reaktor und mindestens eine Destillationskolonne umfassen, und einer sich an RL anschließenden Abtrennstufe A1, die mindestens eine Destillationskolonne DKA1 umfasst, wobei
ein Einsatzgemisch, welches zumindest Butene als Eduktolefine enthält, in dem mindestens einen Reaktor der ersten Reaktionsstufe R1 unter Verwendung eines heterogenen Katalysators einer Oligomerisierung unterworfen wird und das aus dem mindestens einen Reaktor erhaltene Reaktionsgemisch in der mindestens einen Destillationskolonne der ersten Reaktionsstufe R1 destilliert wird, wodurch ein Reststrom RS1, der zumindest nicht umgesetzte Butene enthält, und ein Oligomerisat O1, das zumindest die gebildeten Oligomere enthält, anfallen,
RS1 zumindest teilweise zu einem Reaktor einer nachfolgenden Reaktionsstufe geführt wird;
ein Reststrom aus einer vorherigen Reaktionsstufe in dem mindestens einen Reaktor der letzten Reaktionsstufe RL unter Verwendung eines heterogenen Katalysators einer Oligomerisierung unterworfen wird und das aus dem mindestens einen Reaktor erhaltene Reaktionsgemisch in der mindestens einen Destillationskolonne der letzten Reaktionsstufe RL destilliert wird, wodurch ein Reststrom RSL, der zumindest nicht umgesetzte Butene enthält, und ein Oligomerisat OL, das zumindest die gebildeten Oligomere enthält, anfallen;
OL wird zumindest teilweise zur Destillationskolonne DKA1 der sich anschließenden Abtrennstufe A1 geleitet und dort destilliert, wodurch ein Dimerstrom D1, der zu mindestens 90 Gew.-% aus C8-Oligomeren besteht, und ein Sumpfstrom S1, der zumindest C12-Oligomere und höhere Oligomere der eingesetzten Butene enthält, anfallen;
der Dimerstrom D1 am Kopf der DKA1 anfällt, kondensiert wird und anschließend teilweise als Rücklauf zur DKA1 zurückgeführt und teilweise aus dem Verfahren ausgeschleust wird,
die bei der Kondensation des Dimerstroms D1 entstehende Energie und die im mindestens einen Reaktor der ersten Reaktionsstufe R1 entstehende Wärmeenergie zumindest teilweise auf einen Wärmeträger W1 übertragen werden, wodurch ein erwärmter Wärmeträger W1.1 entsteht; und
Energie vom erwärmten Wärmeträger W1.1 auf einen weiteren Wärmeträger W2 übertragen wird, wodurch ein erwärmter Wärmeträger W2.1 entsteht, der zunächst verdichtet und/oder erwärmt wird und über den anschließend mithilfe einer ein- oder mehrstufigen Verdichtung ein Dampfstrom erzeugt wird, mit dem zumindest die Destillationskolonnen DKA1 und DKR1 beheizt werden.

Das erfindungsgemäße Verfahren umfasst mindestens zwei Reaktionsstufe R1 und RL. Die Reaktionsstufe R1 ist die erste Reaktionsstufe und mit RL ist die letzte Reaktionsstufe gemeint. In einer bevorzugten Ausführungsform der vorliegenden Erfindung ist zwischen der Reaktionsstufe R1 und der Reaktionsstufe RL mindestens eine weitere Reaktionsstufe, die ebenfalls mindestens einen Reaktor und mindestens eine Destillationskolonne umfasst, vorhanden. In einer weiterhin bevorzugten Ausführungsform der vorliegenden Erfindung sind zwischen der Reaktionsstufe R1 und der Reaktionsstufe RL mindestens zwei weitere Reaktionsstufen, die jeweils mindestens einen Reaktor und mindestens eine Destillationskolonne umfassen, vorhanden. In einer besonders bevorzugten Ausführungsform umfasst das Verfahren zur Oligomerisierung maximal fünf Reaktionsstufen, d. h. zwischen der Reaktionsstufe R1 und der Reaktionsstufe RL sind mindestens drei weitere Reaktionsstufen, die jeweils mindestens einen Reaktor und mindestens eine Destillationskolonne umfassen, vorhanden. Es ist in allen Fällen möglich, dass eine der Reaktionsstufen mehrere Reaktoren umfasst, während in einer vorherigen oder nachfolgenden Reaktionsstufe nur ein Reaktor vorhanden ist.

Demzufolge kann das erfindungsgemäße Verfahren in mindestens einer weiteren Reaktionsstufe R2 durchgeführt werden, wobei die Reaktionsstufe R2 mindestens einen Reaktor und mindestens eine Destillationskolonne DKR2 umfasst. Dabei ist es bevorzugt, wenn der für die Beheizung der Destillationskolonnen DKA1 und DKR1 verwendete Dampfstrom auch zur Beheizung der Destillationskolonne DKR2 eingesetzt wird. Es wäre dadurch möglich, dass dann keine zusätzliche extern zu beziehende Energiequelle genutzt werden muss.

An der DKR2 würde im vorgenannten Fall ein Reststrom RS2, der zumindest die nicht umgesetzten Eduktolefine enthält, und ein Oligomerisat O2 anfallen, das zumindest die gebildeten Oligomere enthält. RS2 kann dann zumindest teilweise zur nachfolgenden Reaktionsstufe und gegebenenfalls zumindest teilweise zum mindestens einen Reaktor der mindestens einen Reaktionsstufe R2 zurückgeführt werden. Das Oligomerisat O2 kann zur Destillationskolonne DKRL der letzten Reaktionsstufe RL und/oder zur Destillationskolonne DKA1 der sich anschließende Abtrennstufe A1 geleitet werden, vorzugsweise wird das Oligomerisat zur Destillationskolonne DKRL der letzten Reaktionsstufe RL.

Zusätzlich zur Reaktionsstufe R2 kann auch eine weitere Reaktionsstufe R3 vorliegen. In diesem Fall wird das erfindungsgemäße Verfahren in mindestens einer weiteren Reaktionsstufe R3 durchgeführt, wobei die Reaktionsstufe R3 mindestens einen Reaktor und mindestens eine Destillationskolonne DKR3 umfasst. Dabei ist es bevorzugt, wenn der für die Beheizung der Destillationskolonnen DKA1, DKR1 und DKR2 verwendete Dampfstrom auch zur Beheizung der Destillationskolonne DKR3 eingesetzt wird. Es wäre dadurch möglich, dass dann keine zusätzliche extern zu beziehende Energiequelle genutzt werden muss.

An der DKR3 würde im vorgenannten Fall ein Reststrom RS3, der zumindest die nicht umgesetzten Eduktolefine enthält, und ein Oligomerisat O3 anfallen, das zumindest die gebildeten Oligomere enthält. RS3 kann dann zumindest teilweise zur nachfolgenden Reaktionsstufe und gegebenenfalls zum mindestens einen Reaktor der mindestens einen Reaktionsstufe R3 zurückgeführt werden. Das Oligomerisat O3 kann zur Destillationskolonne DKRL der letzten Reaktionsstufe RL und/oder zur Destillationskolonne DKA1 der sich anschließende Abtrennstufe A1 geleitet werden.

Die Oligomerisierung von Olefinen ist eine exotherme Reaktion, also eine Reaktion, bei der Wärme freigesetzt wird. Die freigesetzte Wärme bei der Oligomerisierung von Butenen entsteht dabei vornehmlich bei der Isomerisierung von 1-Buten zu cis- oder trans-2-Buten. Der oder die Reaktoren in den jeweiligen Reaktionsstufen des erfindungsgemäßen Verfahrens können isotherm oder adiabat betrieben werden, vorzugsweise isotherm. In einer bevorzugten Ausführungsform wird bzw. werden nur der oder die Reaktoren der letzten Reaktionsstufe adiabat betrieben, während alle anderen Reaktoren der vorherigen Reaktionsstufe(n) isotherm betrieben werden, insbesondere aktiv gekühlt werden. Dabei kann ein dem Fachmann bekanntes Kühlmedium, beispielsweise Kühlwasser, eingesetzt werden. Isotherm bedeutet im Rahmen der vorliegenden Erfindung, dass der Temperaturanstieg im Reaktor trotz Kühlens 10 K nicht überschreiten. Dies entspricht einer isothermen Betriebsweise der Reaktoren. Bezogen auf eine Kühlleistung von 100% für den oder die Reaktor(en) in der ersten Reaktionsstufe, beträgt die Kühlleistung in dem oder den Reaktor(en) der nachfolgenden Reaktionsstufen weniger als 100%, aber außer in der letzten Reaktionsstufe nicht 0%.

Das Einsatzgemisch, welches zumindest die eingesetzten Olefine enthält, werden erfindungsgemäß in dem mindestens einen Reaktor der ersten Reaktionsstufe R1 unter Verwendung eines heterogenen Katalysators einer Oligomerisierung unterworfen.

Als der mindestens eine Reaktor in der ersten Reaktionsstufe R1 können alle dem Fachmann bekannten Reaktoren eingesetzt werden, die für die Oligomerisierung geeignet sind, bspw. Rohrreaktoren, Festbettreaktoren, Rohrbündelreaktoren, Settler-Riser-Reaktoren oder Slurry-Reaktoren. Bevorzugt sind Rohrreaktoren und/oder Rohrbündelreaktoren. Sofern die Reaktionsstufe mehrere Reaktoren aufweist, können die Reaktoren gleich oder verschieden voneinander sein. Die Reaktoren in einer Reaktionsstufe können auch in ihrem Aufbau oder ihrer Ausgestaltung variieren. Der erste Reaktor in einer Reaktionsstufe kann beispielsweise ein größeres Volumen aufweisen als der nachfolgende Reaktor in der gleichen Reaktionsstufe. Ebenso ist es möglich, dass die Reaktoren in den einzelnen Reaktionsstufen gleich oder verschieden voneinander sein. Auch dabei ist es möglich, dass die Reaktoren in den einzelnen Reaktionsstufen in ihrem Aufbau oder ihrer Ausgestaltung unterschiedlich sind. Der Reaktor in der ersten Reaktionsstufe kann beispielsweise ein größeres Volumen aufweisen als ein oder alle Reaktoren in den nachfolgenden Reaktionsstufen.

Erfindungsgemäß bevorzugt ist, dass die Reaktionsstufe R1 mindestens einen weiteren Reaktor aufweist, der zum ersten Reaktor parallel oder in Reihe geschaltet wird, vorzugsweise in Reihe geschaltet vorliegt. Der Austrag des ersten Reaktors geht dabei vorzugsweise ohne vorherige Abtrennung der Oligomere in den zweiten Reaktor. Es ist deshalb bevorzugt, dass im ersten Reaktor kein Vollumsatz der Eduktolefine gefahren wird. Die bei der Oligomerisierung entstehende Reaktionswärme des zweiten Reaktors wird vorzugsweise ebenfalls auf den Wärmeträger W1 übertragen.

Der eine oder die Reaktoren der Reaktionsstufe R1 enthält jeweils einen heterogenen Katalysator zur Durchführung der Oligomerisierung. Dabei kann grundsätzlich jeder für die Oligomerisierung geeignete Katalysator eingesetzt werden. Entsprechende Katalysatoren sind dem Fachmann aus der Literatur bekannt. Der verwendete Katalysator liegt dabei insbesondere in Form eines Granulats, eines Extrudats oder in Tablettenform vor.

Der heterogene Katalysator in dem mindestens einen Reaktor R1 umfasst vorzugsweise eine Nickelverbindung, vorzugsweise Nickeloxid, auf einem Alumosilicat-Trägermaterial. Weiterhin bevorzugt enthält der heterogene Katalysator aber, bezogen auf die Gesamtzusammensetzung des Oligomerisierungskatalysators, weniger als 0,5 Gew.-%, noch bevorzugter weniger als 0,1 Gew.-%, besonders bevorzugt weniger als 0,01 Gew.-% Titandioxid und/oder Zirkoniumdioxid. Das Trägermaterial kann ein amorphes, mesoporöses Alumosilicat, ein kristallines, mikroporöses Alumosilicat oder ein Alumosilicat, welches amorphe und kristalline Phasen aufweist, sein. Mit "amorph" im Sinne der vorliegenden Erfindung ist die Eigenschaft eines Feststoffes gemeint, die daraus folgt, dass der Feststoff im Gegensatz zu kristallinen Feststoffen keine Kristallstruktur, d. h. keine Fernordnung, aufweist.

Der heterogene Katalysator weist vorzugsweise eine Zusammensetzung von 15 bis 40 Gew.-%, vorzugsweise 15 bis 30 Gew.-% NiO, 5 bis 30 Gew.-% Al₂O₃, 55 bis 80 Gew.-% SiO₂ und 0,01 bis 2,5 Gew.-%, vorzugsweise 0,05 bis 2 Gew.-% eines Alkalimetalloxids, vorzugsweise Natriumoxid, auf. Die Angaben beziehen sich auf eine Gesamtzusammensetzung von 100 Gew.-%. Der Oligomerisierungskatalysator ist vorzugsweise im Wesentlichen frei von Titandioxid und Zirkoniumdioxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und Zirkoniumdioxid in seiner Gesamtzusammensetzung.

Die Herstellung eines Oligomerisierungskatalysators kann nach den bekannten Verfahren des Imprägnierens, wobei das Trägermaterial mit einer Lösung einer Übergangsmetallverbindung, insbesondere eine Nickelverbindung, beaufschlagt und danach kalziniert wird, oder Co-Fällung, bei der die gesamte Katalysatorzusammensetzung aus einer einzigen, zumeist wässrigen Lösung ausgefällt wird, erfolgen. Der Oligomerisierungskatalysator kann auch nach anderen, dem Fachmann geläufigen, Verfahren hergestellt werden. Bei der Herstellung kann auch eine hydrothermale Behandlung erfolgen, bei dem der noch feuchte Katalysator bei erhöhter Temperatur gehalten wird, bevor der Katalysator einer Trocknung unterworfen wird.

Die Oligomerisierung kann in einem oder allen Reaktor(en) der letzten Reaktionsstufe RL bei einer Temperatur im Bereich von 50 bis 200 °C, vorzugsweise 60 bis 180 °C, bevorzugt im Bereich von 60 bis 130°C durchgeführt wird. Der Druck kann jeder der vorhandenen Reaktionsstufen von 10 bis 70 bar, bevorzugt von 20 bis 55 bar betragen. In einer bevorzugten Ausführungsform der vorliegenden Erfindung wird die Oligomerisierung in jeder Reaktionsstufe in flüssiger Phase durchgeführt. Sofern die Oligomerisierung in flüssiger Phase erfolgen soll, müssen die Parameter Druck und Temperatur hierfür so gewählt werden, dass das Einsatzgemisch (die eingesetzten Olefine oder Olefingemische) in flüssiger Phase vorliegt.

Bei der Oligomerisierung in dem mindestens einen Reaktor der Reaktionsstufe R1 entsteht ein Reaktionsgemisch, welches dem mindestens einen Reaktor entnommen wird. Das aus dem mindestens einen Reaktor erhaltene Reaktionsgemisch wird in der mindestens einen Destillationskolonne DKR1 der ersten Reaktionsstufe R1 destilliert, wodurch ein Reststrom RS1, der zumindest nicht umgesetzte Olefine enthält, und ein Oligomerisat O1, das zumindest die gebildeten Oligomere enthält, anfallen. Durch die Destillation wird demzufolge zumindest ein Teil der gebildeten Oligomere von den nicht umgesetzten Olefinen abgetrennt. Die für die Trennung des Reaktionsgemisches notwendige Energie wird dabei insbesondere durch mindestens einen Sumpfverdampfer eingebracht.

Als Sumpfverdampfer werden erfindungsgemäß Verdampfer bezeichnet, die den Sumpf der Destillationskolonne beheizen. Ein derartiger Sumpfverdampfer ist üblicherweise außerhalb der jeweiligen Destillationskolonne angeordnet. Da in Sumpfverdampfern Energie, insbesondere Wärme, von einem Strom auf einen anderen übertragen wird, sind sie Wärmeüberträger. Über einen Abzug am Sumpf der Destillationskolonne wird der zu verdampfende Strom abgezogen und dem Sumpfverdampfer zugeführt. Über mindestens einen Zulauf wird der verdampfte Strom gegebenenfalls mit einem Restanteil Flüssigkeit wieder in die jeweilige Destillationskolonne im Bereich des Sumpfs zurückgeführt.

Geeignete Verdampfer, die als Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Neben den genannten kann aber auch jede beliebige andere, dem Fachmann bekannte Verdampferbauart, die sich zum Einsatz an einer Destillationskolonne eignet, eingesetzt werden.

Es ist erfindungsgemäß bevorzugt, dass das Reaktionsgemisch vorgewärmt wird, bevor es in die Destillationskolonne DKR1 eingeleitet wird. Das hat den Vorteil, dass nicht die gesamte Energie über den mindestens einen Sumpfverdampfer eingebracht werden muss. Außerdem ermöglicht die Vorwärmung eine Erhöhung der Kapazität, weil die hydraulische Belastung in der Destillationskolonne verringert wird.

Die Vorwärmung des Reaktionsgemisches erfolgt vorzugsweise in einem Wärmetauscher. Die Vorwärmung kann dabei mit dem Fachmann bekannten Wärmetauschern durchgeführt werden. Geeignete Verdampfer, die als Wärmetauscher eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Neben den genannten kann aber auch jede beliebige andere, dem Fachmann bekannte Verdampferbauart, die sich zum Einsatz für die gewünschte Vorwärmung eignet, eingesetzt werden.

Als die mindestens eine Destillationskolonne DKR1 für die Destillation des Reaktionsgemischs können beliebige, dem Fachmann bekannte Destillationskolonnen eingesetzt werden. Bevorzugt enthält die Destillationskolonne der ersten Reaktionsstufe R1 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne der ersten Reaktionsstufe R1 möglichst gering bleibt.

In einer besonders bevorzugten Ausführungsform der vorliegenden Erfindung umfasst die Destillationskolonne der ersten Reaktionsstufe R1 eine Vielzahl von Böden, vorzugsweise zwischen 5 und 100 Böden, weiterhin bevorzugt zwischen 10 und 50 Böden.

Die Bedingungen der Destillation, also beispielsweise Temperatur und Druck, sind üblicherweise durch den Aufbau (Höhe Kolonne, Anzahl der Böden, Art der Böden bzw. Packung, Abstände usw.) festgelegt. Während des Betriebs können die Trenneigenschaften der Destillation noch über die Temperaturverteilung und/oder die Wärmezufuhr in der Kolonne und den Rücklauf im Destillat gesteuert werden. Ebenso kann die Trenneigenschaft durch die Veränderung des Drucks in einem gewissen Rahmen eingestellt werden. Die genauen Einstellungen lassen sich daher nicht übergeordnet und unabhängig von dem Aufbau der Destillationskolonne definieren. Das ist dem Fachmann bekannt. In einer bevorzugten Ausführungsform liegt der Druck bei der Destillation im Bereich von 1 bis 6 bar absolut, besonders bevorzugt im Bereich von 2 bis 5 bar absolut. Die Temperatur am Kopf der Destillationskolonne liegt weiterhin bevorzugt im Bereich von 15 bis 60 °C, besonders bevorzugt im Bereich von 25 bis 50 °C.

Durch die Destillation des Reaktionsgemisches fallen ein Reststrom RS1, der zumindest nicht umgesetzte Olefine enthält, und ein Oligomerisat O1, das zumindest die gebildeten Oligomere enthält, an. Der Reststrom RS1 wird in aller Regel am Kopf der Destillationskolonne DKR1 anfallen. Das Oligomerisat O1 fällt dann am Sumpf der Destillationskolonne DKR1 an.

Der Reststrom RS1 wird zumindest teilweise zu einem Reaktor einer nachfolgenden Reaktionsstufe geführt. Die nachfolgende Reaktionsstufe kann die Reaktionsstufe RL sein. Dann liegen insgesamt nur zwei Reaktionsstufen vor. Die nachfolgende Reaktionsstufe kann aber auch eine zwischen der Reaktionsstufe R1 und der Reaktionsstufe RL vorhandene Reaktionsstufe sein. In diesem Fall liegen mindestens drei Reaktionsstufen vor. Es sind auch Ausführungsformen möglich, bei der zwischen der Reaktionsstufe R1 und der Reaktionsstufe RL mehr als zwei Reaktionsstufen vorhanden sind. Es ist erfindungsgemäß bevorzugt, dass zwischen der Reaktionsstufe R1 und der Reaktionsstufe RL mindestens eine weitere Reaktionsstufe, die mindestens einen Reaktor und mindestens eine Destillationskolonne umfasst, vorhanden ist.

Es ist weiterhin bevorzugt, dass der Reststrom RS1 zumindest teilweise zum mindestens einen Reaktor der Reaktionsstufe R1 zurückgeführt wird. Durch diesen Rücklauf an den Reststrom kann das Einsatzgemisch verdünnt werden.

In einer bevorzugten Ausführungsform der vorliegenden Erfindung wird der Reststrom RS1 in mindestens zwei Teilströme RS1a und RS1b aufgetrennt. Der Teilstrom RS1b kann dann zum Reaktor der ersten Reaktionsstufe R1 zurückgeführt werden. Es ist weiterhin bevorzugt, dass der Teilstrom RS1a verdichtet wird, wodurch ein gegenüber dem Reststrom RS1 verdichteter Strom VR1 entsteht und Energie von dem verdichteten Strom VR1 zur Vorwärmung des Reaktionsgemisches vor der DKR1 und/oder zur Vorwärmung des Einsatzgemischs vor der Oligomerisierung eingesetzt wird. Die Vorwärmung kann also auch mittels wärmeintegrativer Maßnahmen, hier der Brüdenverdichtung, was die Zufuhr von externer Energie in Form von Heizdampf unnötig macht. Hier muss lediglich elektrische Energie für die Verdichtung eingesetzt werden.

Das Verdichten des Teilstroms RS1a kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Teilstrom RS1a verdichtet werden soll.

Als Verdichter im erfindungsgemäßen Verfahren, insbesondere zum Verdichten Teilstroms RS1a, eignet sich jeder beliebige, dem Fachmann bekannte Verdichter, bevorzugt mechanische Verdichter, mit dem sich auch Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Es ist bereits erwähnt worden, dass in einer bevorzugten Ausführungsform bei dem Verfahren mindestens eine zweite Reaktionsstufe R2 vorliegt, wobei in dem mindestens einen Reaktor ein Reaktionsgemisch anfällt, dass in der Destillationskolonne DKR2 aufgetrennt wird. Dabei ist es besonders bevorzugt, dass Energie von dem verdichteten Strom VR1 auch zur Vorwärmung des Reaktionsgemisches vor der DKR2 eingesetzt wird.

Nach der Vorwärmung kann der Teilstrom RS1a zumindest teilweise kondensiert und anschließend zumindest teilweise als Einsatzgemisch zum mindestens einen Reaktor der zweiten Reaktionsstufe R2 geleitet werden.

Das Oligomerisat O1 enthält die gebildeten Oligomere. In einer bevorzugten Ausführungsform wird das Oligomerisat O1 zumindest teilweise, vorzugsweise vollständig zur Destillationskolonne der letzten Reaktionsstufe RL geleitet. Dort können alle Oligomerisate gesammelt und anschließend gemeinsam zur Abtrennstufe A1 geführt werden. Zudem können dort noch Reste an nicht umgesetzten Olefinen abgetrennt werden.

Unabhängig davon wie viele Reaktionsstufen vorhanden sind, wird der Reststrom aus der vorherigen Reaktionsstufe zur letzten Reaktionsstufe RL geführt. Das kann zumindest ein Teil des Reststroms RS1 aus der ersten Reaktionsstufe R1 sein, könnte aber auch ein anderer Reststrom sein, sofern zumindest eine weitere Reaktionsstufe zwischen R1 und RL vorhanden ist. Insofern wird ein Reststrom aus einer vorherigen Reaktionsstufe zum mindestens einen Reaktor der letzten Reaktionsstufe RL geführt. Dieser Reststrom kann der Reststrom RS1 ein, wenn zwischen den beiden Reaktionsstufen R1 und RL keine weitere Reaktionsstufe vorhanden ist. Sofern mindestens eine Reaktionsstufe zwischen der Reaktionsstufe R1 und der Reaktionsstufe RL vorhanden ist wird der Reststrom aus einer dieser Reaktionsstufen, der vorletzten Reaktionsstufe zur letzten Reaktionsstufe RL geführt.

Der zugeführte Reststrom wird erfindungsgemäß in dem mindestens einen Reaktor der letzten Reaktionsstufe RL unter Verwendung eines heterogenen Katalysators einer Oligomerisierung unterworfen.

Als der mindestens eine Reaktor in der ersten Reaktionsstufe RL können alle dem Fachmann bekannten Reaktoren eingesetzt werden, die für die Oligomerisierung geeignet sind, bspw. Rohrreaktoren, Festbettreaktoren, Rohrbündelreaktoren, Settler-Riser-Reaktoren oder Slurry-Reaktoren. Bevorzugt sind Rohrreaktoren und/oder Rohrbündelreaktoren. Sofern die Reaktionsstufe mehrere Reaktoren aufweist, können die Reaktoren gleich oder verschieden voneinander sein. Die Reaktoren in einer Reaktionsstufe können auch in ihrem Aufbau oder ihrer Ausgestaltung variieren. Der erste Reaktor in einer Reaktionsstufe kann beispielsweise ein größeres Volumen aufweisen als der nachfolgende Reaktor in der gleichen Reaktionsstufe. Ebenso ist es möglich, dass die Reaktoren in den einzelnen Reaktionsstufen gleich oder verschieden voneinander sein. Auch dabei ist es möglich, dass die Reaktoren in den einzelnen Reaktionsstufen in ihrem Aufbau oder ihrer Ausgestaltung unterschiedlich sind. Der Reaktor in der ersten Reaktionsstufe kann beispielsweise ein größeres Volumen aufweisen als ein oder alle Reaktoren in den nachfolgenden Reaktionsstufen.

Erfindungsgemäß bevorzugt ist, dass die Reaktionsstufe R1 mindestens einen weiteren Reaktor aufweist, der zum ersten Reaktor parallel oder in Reihe geschaltet wird, vorzugsweise in Reihe geschaltet vorliegt.

Der Austrag des ersten Reaktors geht dabei vorzugsweise ohne vorherige Abtrennung der Oligomere in den zweiten Reaktor. Es ist deshalb bevorzugt, dass im ersten Reaktor kein Vollumsatz der Eduktolefine gefahren wird. Die in diesem weiteren Reaktor entstehende Reaktionswärme kann ebenfalls über einen Wärmeträger integriert werden, wie dies bereits für den ersten Reaktor beschrieben worden ist.

Der eine oder die Reaktoren der Reaktionsstufe RL enthält jeweils einen heterogenen Katalysator zur Durchführung der Oligomerisierung. Dabei kann grundsätzlich jeder für die Oligomerisierung geeignete Katalysator eingesetzt werden. Entsprechende Katalysatoren sind dem Fachmann aus der Literatur bekannt. Der verwendete Katalysator liegt dabei insbesondere in Form eines Granulats, eines Extrudats oder in Tablettenform vor.

Der heterogene Katalysator in dem mindestens einen Reaktor der Reaktionsstufe RL umfasst vorzugsweise eine Nickelverbindung, vorzugsweise Nickeloxid, auf einem Alumosilicat-Trägermaterial. Weiterhin bevorzugt enthält der heterogene Katalysator aber, bezogen auf die Gesamtzusammensetzung des Oligomerisierungskatalysators, weniger als 0,5 Gew.-%, noch bevorzugter weniger als 0,1 Gew.-%, besonders bevorzugt weniger als 0,01 Gew.-% Titandioxid und/oder Zirkoniumdioxid. Das Trägermaterial kann ein amorphes, mesoporöses Alumosilicat, ein kristallines, mikroporöses Alumosilicat oder ein Alumosilicat, welches amorphe und kristalline Phasen aufweist sein. Mit "amorph" im Sinne der vorliegenden Erfindung ist die Eigenschaft eines Feststoffes gemeint, die daraus folgt, dass der Feststoff im Gegensatz zu kristallinen Feststoffen keine Kristallstruktur, d. h. keine Fernordnung, aufweist.

Der heterogene Katalysator in dem mindestens einen Reaktor der Reaktionsstufe RL weist vorzugsweise eine Zusammensetzung von 15 bis 40 Gew.-%, vorzugsweise 15 bis 30 Gew.-% NiO, 5 bis 30 Gew.-% Al₂O₃, 55 bis 80 Gew.-% SiO₂ und 0,01 bis 2,5 Gew.-%, vorzugsweise 0,05 bis 2 Gew.-% eines Alkalimetalloxids, vorzugsweise Natriumoxid, auf. Die Angaben beziehen sich auf eine Gesamtzusammensetzung von 100 Gew.-%. Der Oligomerisierungskatalysator ist vorzugsweise im Wesentlichen frei von Titandioxid und Zirkoniumdioxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und Zirkoniumdioxid in seiner Gesamtzusammensetzung.

Die Herstellung eines Oligomerisierungskatalysators kann nach den bekannten Verfahren des Imprägnierens, wobei das Trägermaterial mit einer Lösung einer Übergangsmetallverbindung, insbesondere eine Nickelverbindung, beaufschlagt und danach kalziniert wird, oder Co-Fällung, bei der die gesamte Katalysatorzusammensetzung aus einer einzigen, zumeist wässrigen Lösung ausgefällt wird, erfolgen. Der Oligomerisierungskatalysator kann auch nach anderen, dem Fachmann geläufigen, Verfahren hergestellt werden. Bei der Herstellung kann auch eine hydrothermale Behandlung erfolgen, bei dem der noch feuchte Katalysator bei erhöhter Temperatur gehalten wird, bevor der Katalysator einer Trocknung unterworfen wird.

Die Oligomerisierung wird in dem oder den Reaktor(en) der letzten Reaktionsstufe vorzugsweise bei einer Temperatur im Bereich von 50 bis 200 °C, vorzugsweise 60 bis 180 °C, bevorzugt im Bereich von 60 bis 130°C durchgeführt wird. Der Druck kann dabei im Bereich von 10 bis 70 bar, bevorzugt von 20 bis 55 bar liegen. In einer bevorzugten Ausführungsform der vorliegenden Erfindung wird die Oligomerisierung in jeder Reaktionsstufe in flüssiger Phase durchgeführt. Sofern die Oligomerisierung in flüssiger Phase erfolgen soll, müssen die Parameter Druck und Temperatur hierfür so gewählt werden, dass das Einsatzgemisch (die eingesetzten Olefine oder Olefingemische) in flüssiger Phase vorliegt.

Bei der Oligomerisierung in dem mindestens einen Reaktor der Reaktionsstufe RL entsteht ein Reaktionsgemisch, welches dem mindestens einen Reaktor entnommen wird. Das aus dem mindestens einen Reaktor erhaltene Reaktionsgemisch wird in der mindestens einen Destillationskolonne DKRL der letzten Reaktionsstufe RL destilliert, wodurch ein Reststrom RSL, der zumindest nicht umgesetzte Olefine enthält, und ein Oligomerisat OL, das zumindest die gebildeten Oligomere enthält, anfallen. Durch die Destillation wird demzufolge zumindest ein Teil der gebildeten Oligomere von den nicht umgesetzten Olefinen abgetrennt. Zusätzlich zum Reaktionsgemisch können noch die Oligomerisate aus den vorherigen Reaktionsstufen, beispielsweise das Oligomerisat O1, in der Destillationskolonne DKRL aufgearbeitet werden. Die für die Trennung des Reaktionsgemisches notwendige Energie wird dabei insbesondere durch mindestens einen Sumpfverdampfer eingebracht.

Als Sumpfverdampfer werden erfindungsgemäß Verdampfer bezeichnet, die den Sumpf der Destillationskolonne beheizen. Ein derartiger Sumpfverdampfer ist üblicherweise außerhalb der jeweiligen Destillationskolonne angeordnet. Da in Sumpfverdampfern Energie, insbesondere Wärme, von einem Strom auf einen anderen übertragen wird, sind sie Wärmeüberträger. Über einen Abzug am Sumpf der Destillationskolonne wird der zu verdampfende Strom abgezogen und dem Sumpfverdampfer zugeführt. Über mindestens einen Zulauf wird der verdampfte Strom gegebenenfalls mit einem Restanteil Flüssigkeit wieder in die jeweilige Destillationskolonne im Bereich des Sumpfs zurückgeführt.

Geeignete Verdampfer, die als Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Neben den genannten kann aber auch jede beliebige andere, dem Fachmann bekannte Verdampferbauart, die sich zum Einsatz an einer Destillationskolonne eignet, eingesetzt werden.

Es ist erfindungsgemäß bevorzugt, dass das Reaktionsgemisch vorgewärmt wird, bevor es in die Destillationskolonne DKRL eingeleitet wird. Das hat den Vorteil, dass nicht die gesamte Energie über den mindestens einen Sumpfverdampfer eingebracht werden muss. Außerdem ermöglicht die Vorwärmung eine Erhöhung der Kapazität, weil die hydraulische Belastung in der Destillationskolonne verringert wird.

Die Vorwärmung des Reaktionsgemisches erfolgt vorzugsweise in einem Wärmetauscher. Die Vorwärmung kann dabei mit dem Fachmann bekannten Wärmetauschern durchgeführt werden. Geeignete Verdampfer, die als Wärmetauscher eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Neben den genannten kann aber auch jede beliebige andere, dem Fachmann bekannte Verdampferbauart, die sich zum Einsatz für die gewünschte Vorwärmung eignet, eingesetzt werden.

Als die mindestens eine Destillationskolonne DKRL für die Destillation des Reaktionsgemischs aus dem Reaktor der letzten Reaktionsstufe RL können beliebige, dem Fachmann bekannte Destillationskolonnen eingesetzt werden. Bevorzugt enthält die Destillationskolonne DKRL der letzten Reaktionsstufe RL Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne der ersten Reaktionsstufe RL möglichst gering bleibt.

In einer besonders bevorzugten Ausführungsform der vorliegenden Erfindung umfasst die Destillationskolonne DKRL eine Vielzahl von Böden, vorzugsweise zwischen 5 und 100 Böden, weiterhin bevorzugt zwischen 10 und 50 Böden.

Die Bedingungen der Destillation, also beispielsweise Temperatur und Druck, sind üblicherweise durch den Aufbau (Höhe Kolonne, Anzahl der Böden, Art der Böden bzw. Packung, Abstände usw.) festgelegt. Während des Betriebs können die Trenneigenschaften der Destillation noch über die Temperaturverteilung und/oder die Wärmezufuhr in der Kolonne und den Rücklauf im Destillat gesteuert werden. Ebenso kann die Trenneigenschaft durch die Veränderung des Drucks in einem gewissen Rahmen eingestellt werden. Die genauen Einstellungen lassen sich daher nicht übergeordnet und unabhängig von dem Aufbau der Destillationskolonne definieren. Das ist dem Fachmann bekannt. Der Druck bei der Destillation liegt vorzugsweise im Bereich von 1 bis 6 bar absolut, vorzugsweise im Bereich von 2 bis 5 bar absolut.

Durch die Destillation des Reaktionsgemisches sowie der optional vorhandenen Oligomerisate aus vorherigen Stufen fallen ein Reststrom RSL, der zumindest nicht umgesetzte Olefine enthält, und ein Oligomerisat OL, das zumindest die gebildeten Oligomere enthält, an. Der Reststrom RSL wird in aller Regel am Kopf der Destillationskolonne DKRL anfallen. Das Oligomerisat OL fällt dann am Sumpf der Destillationskolonne DKRL an.

Der Reststrom RL kann zumindest teilweise zu einem oder mehreren Reaktoren der gleichen Reaktionsstufe oder der vorherigen Reaktionsstufe(n) zurückgeführt werden. Es kann dabei auch zumindest ein Teilstrom als Purge entnommen werden, um das Anreichern von inerten Alkanen oder das Anreichern von Verunreinigungen zu verhindern. Wird zumindest ein Teilstrom als Purge entnommen und damit aus dem Verfahren ausgeschleust, kann dieser Teilstrom als Syntheserohstoff für weitere Verfahren (z. B. Hydroformylierung, C-Quelle für Lichtbogen bei der Acetylenherstellung), als Verbrennungsgas oder nach Vollhydrierung zu den Alkanen als Treibgas, als Kochgas o. ä. dienen.

Das Oligomerisat OL enthält die gebildeten Oligomere. Zu den gebildeten Oligomeren gehören in aller Regel zumindest Dimere, Trimere und Tetramere. Höhere Oligomere können jedoch in Kleinstmengen ebenfalls im Oligomerisat vorhanden sein. Das erhaltene Oligomerisat wird deshalb einer weiteren Aufarbeitung in der Abtrennstufe A1 zugeführt, um die Dimere, Trimere und Tetramere voneinander zu trennen.

Das Oligomerisat OL wird also zumindest teilweise zur Destillationskolonne DKA1 der Abtrennstufe A1 geleitet und dort destilliert, wodurch ein Dimerstrom D1, der, bezogen auf das Gesamtgewicht des Dimerstroms D1, zu mindestens 90 Gew.-%, vorzugsweise zu mindestens 95 Gew.-%, besonders bevorzugt zu mindestens 99 Gew.-% aus Dimeren der eingesetzten Olefine besteht, und ein Sumpfstrom S1, der zumindest Trimere und höhere Oligomere der eingesetzten Olefine enthält, anfallen. Vorzugsweise wird das Oligomerisat OL vollständig zur Destillationskolonne DKA1 der Abtrennstufe A1 geleitet.

Der Sumpfstrom S1, der zumindest Trimere und höhere Oligomere der eingesetzten Eduktolefine enthält, kann in einem oder mehreren nachfolgenden Destillationsschritten in die weiteren Oligomere zerlegt werden, die anschließend zu Wertprodukten wie Aldehyden oder Alkoholen umgesetzt werden. Die Oligomere aus dem Dimerstrom D1 können dann jeweils einer weiteren Verarbeitung zugeführt werden, beispielsweise einer Hydroformylierung, einer Alkoxycarbonylierung oder einer Hydrierung. Die Dimere von Butenen, C8-Olefine, können durch Hydroformylierung zu einem Isononanalgemisch, umgesetzt werden. Dieses sononanalgemisch liefert entweder durch Oxidation die entsprechenden Carbonsäuren oder durch Hydrierung ein C9-Alkoholgemisch. Das C9-Carbonsäuren können zur Herstellung von Schmiermitteln oder Sikkative verwendet werden. Das C9-Alkoholgemisch ist Vorstufe für die Herstellung von Weichmachern, insbesondere von Di-isononyl-phthalaten (DINP), DINCH (1,2-Di-isononyl-cyclohexandicarbonsäureester) oder DINCD (1,4-Di-isononyl-cyclohexandicarbonsäureester).

Die für die Trennung des Oligomerisats OL in der Destillationskolonne DKA1 notwendige Energie wird dabei insbesondere durch mindestens einen Sumpfverdampfer eingebracht. Als Sumpfverdampfer werden erfindungsgemäß Verdampfer bezeichnet, die den Sumpf der Destillationskolonne beheizen. Ein derartiger Sumpfverdampfer ist üblicherweise außerhalb der jeweiligen Destillationskolonne angeordnet. Da in Sumpfverdampfern Energie, insbesondere Wärme, von einem Strom auf einen anderen übertragen wird, sind sie Wärmeüberträger. Über einen Abzug am Sumpf der Destillationskolonne wird der zu verdampfende Strom abgezogen und dem Sumpfverdampfer zugeführt. Über mindestens einen Zulauf wird der verdampfte Strom gegebenenfalls mit einem Restanteil Flüssigkeit wieder in die jeweilige Destillationskolonne im Bereich des Sumpfs zurückgeführt.

Geeignete Verdampfer, die als Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Neben den genannten kann aber auch jede beliebige andere, dem Fachmann bekannte Verdampferbauart, die sich zum Einsatz an einer Destillationskolonne eignet, eingesetzt werden.

Als die mindestens eine Destillationskolonne DKA1 für die Destillation des Oligomerisats OL können beliebige, dem Fachmann bekannte Destillationskolonnen eingesetzt werden. Bevorzugt enthält die Destillationskolonne DKA1 der Abtrennstufe A1 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne der ersten Reaktionsstufe RL möglichst gering bleibt.

In einer besonders bevorzugten Ausführungsform der vorliegenden Erfindung umfasst die Destillationskolonne DKA1 eine Vielzahl von Böden, vorzugsweise zwischen 5 und 100 Böden, weiterhin bevorzugt zwischen 10 und 50 Böden.

Die Bedingungen der Destillation, also beispielsweise Temperatur und Druck, sind üblicherweise durch den Aufbau (Höhe Kolonne, Anzahl der Böden, Art der Böden bzw. Packung, Abstände usw.) festgelegt. Während des Betriebs können die Trenneigenschaften der Destillation noch über die Temperaturverteilung und/oder die Wärmezufuhr in der Kolonne und den Rücklauf im Destillat gesteuert werden. Ebenso kann die Trenneigenschaft durch die Veränderung des Drucks in einem gewissen Rahmen eingestellt werden. Die genauen Einstellungen lassen sich daher nicht übergeordnet und unabhängig von dem Aufbau der Destillationskolonne definieren. Das ist dem Fachmann bekannt.

Der Dimerstrom D1, der am Kopf der DKA1 anfällt, wird kondensiert und anschließend teilweise als Rücklauf zur DKA1 zurückgeführt und teilweise aus dem Verfahren ausgeschleust. Die bei der Kondensation des Dimerstroms D1 entstehende Energie und die im mindestens einen Reaktor der ersten Reaktionsstufe R1 entstehende Wärmeenergie zumindest teilweise auf einen Wärmeträger W1 übertragen werden, wodurch ein erwärmter Wärmeträger W1.1 entsteht.

Die Kondensation des Dimerstroms D1 kann in jedem dem Fachmann bekannten Kondensator erfolgen, der wie ein Wärmetauscher ausgestaltet ist, um die Kondensationswärmeenergie auf den flüssigen oder gasförmigen Wärmeträger W1 übertragen zu können. Mögliche Wärmetauscher, die eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. In einer bevorzugten Ausführungsform wird als Wärmetauscher ein Kesselverdampfer eingesetzt, in dessen Rohren der Dimerstrom D1 kondensiert und in dessen Mantelraum der Wärmeträger beheizt wird.

Als Wärmeträger W1 kann in dem erfindungsgemäßen Verfahren jedes dem Fachmann geläufige Arbeitsmedium genutzt werden. Der Wärmeträger W1 wird vorzugsweise aus der Gruppe bestehend aus Wasser; Alkoholen; Alkohol-Wasser-Mischungen; Salz-Wasser-Lösungen; Ammoniak; Mineralölen, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt. Bevorzugt werden Wasser, Methanol, Ethanol, Propanol, n-Pentan, n-Butan, n-Hexan, n-Propan oder Ammoniak als Wärmeträger W1 eingesetzt. Besonders bevorzugt wird Wasser als Wärmeträger W1 eingesetzt.

Wird der Wärmeträger W1 in flüssiger Phase, also ein flüssiger Wärmeträger W1, eingesetzt und ihm Energie, bevorzugt Wärme, zugeführt, wird der Wärmeträger W1 zumindest teilweise verdampft und dadurch ein zumindest teilweise gasförmiger Wärmeträger W1.1 erhalten. Wird der Wärmeträger W1 in gasförmiger Phase, also ein gasförmiger Wärmeträger W1, eingesetzt und ihm Energie, bevorzugt Wärme, zugeführt, wird ebenfalls ein gasförmiger Wärmeträger W1.1 erhalten.

In einer bevorzugten Ausführungsform der vorliegenden Erfindung umfasst die die Abtrennstufe A1 mindestens eine weitere Destillationskolonne DKA2 zur Destillation des Sumpfstroms S1, mit der Trimere von den höheren Oligomeren abgetrennt werden. Die Trimere würden dabei als Strom am Kopf der Destillationskolonne DKA2 anfallen. Die höheren Oligomere fallen dementsprechend im Sumpf der DKA2 an.

Als die mindestens eine Destillationskolonne DKA2 für die Destillation des Sumpfstroms S1 können beliebige, dem Fachmann bekannte Destillationskolonnen eingesetzt werden. Bevorzugt enthält die Destillationskolonne DKA2 der Abtrennstufe A1 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne der ersten Reaktionsstufe RL möglichst gering bleibt.

In einer besonders bevorzugten Ausführungsform der vorliegenden Erfindung umfasst die Destillationskolonne DKA2 eine Vielzahl von Einbauten, vorzugsweise zwischen 5 und 80 Trennstufen, weiterhin bevorzugt zwischen 10 und 40 Trennstufen.

Die Bedingungen der Destillation, also beispielsweise Temperatur und Druck, sind üblicherweise durch den Aufbau (Höhe Kolonne, Anzahl der Böden, Art der Böden bzw. Packung, Abstände usw.) festgelegt. Während des Betriebs können die Trenneigenschaften der Destillation noch über die Temperaturverteilung und/oder die Wärmezufuhr in der Kolonne und den Rücklauf im Destillat gesteuert werden. Ebenso kann die Trenneigenschaft durch die Veränderung des Drucks in einem gewissen Rahmen eingestellt werden. Die genauen Einstellungen lassen sich daher nicht übergeordnet und unabhängig von dem Aufbau der Destillationskolonne definieren. Das ist dem Fachmann bekannt. Der Druck bei der Destillation liegt vorzugsweise im Vakuum, weiterhin bevorzugt im Bereich von 100 bis 800 mbar, weiterhin bevorzugt im Bereich von 200 bis 700 mbar, besonders bevorzugt 300 bis 500 mbar.

Der bei der DKA2 anfallende Kopfstrom wird vorzugsweise kondensiert und anschließend vorzugsweise teilweise als Rücklauf zur DKA2 zurückgeführt und teilweise aus dem Verfahren ausgeschleust. Die bei der Kondensation Kopfstroms der DKA2 entstehende Energie wird besonders bevorzugt zusätzlich zumindest teilweise auf einen Wärmeträger W1 übertragen werden.

Die Energie vom erwärmten Wärmeträger W1.1 wird anschließend auf einen weiteren Wärmeträger W2 übertragen, wodurch ein erwärmter Wärmeträger W2.1 entsteht, der zunächst verdichtet und/oder erwärmt wird und über den danach mithilfe einer ein- oder mehrstufigen Verdichtung ein Dampfstrom erzeugt wird, mit dem zumindest die Destillationskolonnen DKA1 und DKR1 beheizt werden.

Zunächst erfolgt also die Übertragung von Energie, insbesondere Wärmeenergie, vom Wärmeträger W1.1 auf den Wärmeüberträger W2. Als Wärmeträger W2 kann in dem erfindungsgemäßen Verfahren jedes dem Fachmann geläufige Arbeitsmedium genutzt werden. Der Wärmeträger W2 wird vorzugsweise aus der Gruppe bestehend aus Wasser; Alkoholen; Alkohol-Wasser-Mischungen; Salz-Wasser-Lösungen; Ammoniak; Mineralölen, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt. Bevorzugt werden Wasser, Methanol, Ethanol, Propanol, n-Pentan, n-Butan, n-Hexan, n-Propan oder Ammoniak als Wärmeträger W2 eingesetzt. Besonders bevorzugt wird Wasser und/oder Methanol als Wärmeträger W2 eingesetzt.

Wird der Wärmeträger W2 in flüssiger Phase, also ein flüssiger Wärmeträger W2, eingesetzt und ihm Energie, bevorzugt Wärme, zugeführt, wird der Wärmeträger W2 zumindest teilweise verdampft und dadurch ein zumindest teilweise gasförmiger Wärmeträger W2.1 erhalten. Wird der Wärmeträger W2 in gasförmiger Phase, also ein gasförmiger Wärmeträger W2, eingesetzt und ihm Energie, bevorzugt Wärme, zugeführt, wird ebenfalls ein gasförmiger Wärmeträger W2.1 erhalten.

Die Übertragung von Energie von W1.1 auf W2 kann nach dem Fachmann bekannten Verfahren oder mit dem Fachmann bekannten Wärmetauschern durchgeführt werden, beispielsweise den bereits erwähnten Verdampfern, durchgeführt werden. Geeignete Verdampfer, die als Wärmetauscher eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Neben den genannten kann aber auch jede beliebige andere, dem Fachmann bekannte Verdampferbauart eingesetzt werden, sofern damit die gewünschte Energieübertragung gewährleistet ist.

Die Wärmeträger W1 und W2 können grundsätzlich gleich oder verschiedenen voneinander sein. Das bedeutet, dass es grundsätzlich möglich ist, dass der Wärmetauscher W1 und der Wärmetauscher die gleiche Flüssigkeit oder das gleiche Gas sind. Genauso gut ist es möglich, dass die beiden Wärmeträger W1 und W2 unterschiedlich sind. Beide Verfahrensvarianten werden nachfolgend beschrieben.

Der erwärmter Wärmeträger W2.1 wird erfindungsgemäß zunächst verdichtet und/oder erwärmt, um anschließend mithilfe einer ein- oder mehrstufigen Verdichtung einen Dampfstrom erzeugen zu können. Eine Erwärmung kann beispielsweise mittels der bereits beschriebenen Verdampfer erfolgen. Das Verdichten kann auf jede beliebige, dem Fachmann bekannte Art erfolgen, wie es bereits im Vorhinein beschrieben worden ist.

In einer besonders bevorzugten Ausführungsform der vorliegenden Erfindung wird als Wärmeträger W2 Methanol eingesetzt. Durch die Energieübertragung vom erwärmten Wärmeträger W1.1 wird das Methanol auf einen höheren Druck und/oder eine höhere Temperatur gebracht (und als W2.1 bezeichnet) und anschließend ein- oder mehrstufig, vorzugsweise mehrstufig, verdichtet. Durch die sich anschließende Verdichtung entsteht der verdichtete Wärmeträger W2.2, hier Methanol.

Die Verdichtung von Methanol als W2.1 kann zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Wärmeträger W2.1 verdichtet werden soll.

Im vorliegenden Fall wird das Methanol als W2.1 durch die ein- oder mehrstufige Verdichtung zu W2.2 auf einen Druck im Bereich von 3 bis 30 bar, vorzugsweise 5 bis 20 bar, vorzugsweise 7 bis 15 bar gebracht.

Als Verdichter im erfindungsgemäßen Verfahren, insbesondere zum Verdichten von Methanol als dem Wärmeträger W2.1, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

In einer ebenso besonders bevorzugten Ausführungsform der vorliegenden Erfindung wird als Wärmeträger W2 Wasser eingesetzt, das im leichten Vakuum im Bereich zwischen 300 und 800 mbar vorliegt. Durch die Energieübertragung vom erwärmten Wärmeträger W1.1 wird das Wasser zumindest teilweise verdampft (und als W2.1 bezeichnet). Das teilweise verdampfte Wasser als W2.1 wird vorzugsweise über ein ein- oder mehrstufiges, vorzugsweise mehrstufiges Gebläse auf einen höheren Druck gebracht, also verdichtet, und fällt als Schlappdampf an. Durch die beschriebene Verdichtung entsteht der verdichtete Wärmeträger W2.2, hier Schlappdampf. Der entstehende Schlappdampf wird vorzugsweise zur Beheizung bzw. Vorwärmung der Feedströme zu den Destillationskolonnen eingesetzt.

Unabhängig davon welcher Wärmeträger als Wärmeträger W2 eingesetzt wird, wird in jedem Fall ein erwärmter Wärmeträger W2.1 anfallen, der verdichtet wird und als verdichteter Wärmeträger W2.2 anfällt. In einer weiterhin bevorzugten Ausführungsform der vorliegenden Erfindung wird anschließend Energie vom verdichteten Wärmeträger W2.2 auf einen weiteren Wärmeträger W3, vorzugsweise Wasser, übertragen, wodurch ein erwärmter Wärmeträger W3.1 entsteht, der ein- oder mehrstufig, vorzugsweise mehrstufig, verdichtet wird und anschließend als Dampfstrom zur Beheizung zumindest der Destillationskolonnen DKA1 und DKR1, vorzugsweise aller in den Reaktionsstufen und aller in der Abtrennstufe vorhandenen Destillationskolonnen eingesetzt wird. Ferner kann der durch die erfindungsgemäß beschriebene Wärmeintegration erzeugte Dampfstrom auch zusätzlich zur Beheizung der Destillationskolonne DKA2 eingesetzt werden, sofern diese Destillationskolonne vorhanden ist.

Der Wärmeträger W3, vorzugsweise das Wasser, wird ein- oder mehrstufig, vorzugsweise mehrstufig verdichtet. Durch diese ein- oder mehrstufige Verdichtung wird der Wärmeträger W3 auf einen Druck im Bereich von 5 bis 40 bar, vorzugsweise 10 bis 30 bar, vorzugsweise 12 bis 25 bar gebracht und liegt in diesem Bereich als Dampf vor. Sofern eine mehrstufige Verdichtung erfolgt, wird vorzugsweise zwischen den Verdichterstufen Wasserkondensat eingespritzt, um eine Überhitzung bei der Verdichtung zu verhindern.

Die Wärmeträger W1, W2 und W3 werden alle zur Übertragung von Energie, insbesondere Energie in Form von Wärme, eingesetzt. Durch die jeweilige Energieübertragung wird sich die Temperatur und/oder der Druck verringern und es kommt zur zumindest partiellen Kondensation. Die Wärmeträger W1, W2 und W3 bilden dabei vorzugsweise einen eigenen Kreislauf, d. h. die Wärmeträger werden nach der Energieübertragung zurückgeführt, damit wieder Energie eingesammelt werden kann.

Bei der Rückführung der Wärmeträger können diese Wärmeträger eingesetzt werden, um den Wärmeträger im Kreislauf vor der Verdichtung zu erwärmen. Beispielsweise könnte der Wärmeträger W2.2 nach der Energieübertragung noch zusätzlich Energie auf W2.1 vor der Verdichtung übertragen. Dazu können bekannte Wärmetauscher eingesetzt werden.

Die vorliegende Erfindung wird anhand der nachfolgend beschriebenen Zeichnungen beschrieben. Die Zeichnungen stellen lediglich Beispiele für geeignete Ausführungsformen dar, die den Erfindungsgegenstand nicht einschränken.

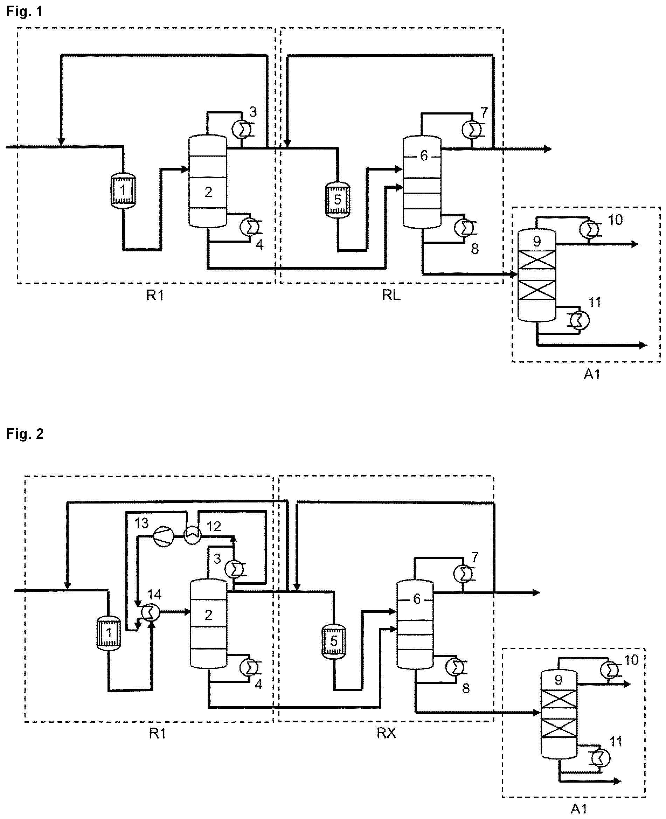

Fig. 1 zeigt eine nicht erfindungsgemäße Verschaltung der Oligomerisierung mit anschließender Auftrennung der Oligomere. Das Einsatzgemisch wird zunächst zur ersten Reaktionsstufe (R1) geführt, wo die Oligomerisierung im Reaktor (1) und die sich anschließende Auftrennung des Reaktionsgemisches in der Destillationskolonne DKR1 (2) erfolgt. Der am Kopf der Destillationskolonne DKR1 (2) anfallende Reststrom wird in einem Kondensator (3) zumindest teilweise kondensiert und anschließend jeweils teilweise zurück zum Reaktor (1) und zur nächsten Reaktionsstufe (RX) geführt. RX wäre in diesem Beispiel RL im Sinne der vorliegenden Erfindung. Das Oligomerisat O1 wird zur Destillationskolonne DKRX (6) geleitet. Die Energie für die Auftrennung in der Destillationskolonne DKR1 (2) wird über einen Sumpfverdampfer (4) eingebracht. Der zur nächsten Reaktionsstufe RX geführte Reststrom wird im Reaktor (5) umgesetzt. Das erhaltene Reaktionsgemisch wird in der Destillationskolonne DKRX (6) aufgetrennt. DKRX entspräche in diesem Beispiel DKRL. Der am Kopf der

Destillationskolonne DKRX (6) anfallende Reststrom wird in einem Kondensator (7) zumindest teilweise kondensiert und anschließend mindestens teilweise zurück zum Reaktor (5) geführt. Die Energie für die Auftrennung in der Destillationskolonne DKRX (6) wird über einen Sumpfverdampfer (8) eingebracht. Das im Sumpf anfallende Oligomerisat OL wird zu Abtrennstufe A1 geleitet, wo die Auftrennung der Oligomere in der Destillationskolonne DKA1 (9) erfolgt. Der Dimerstrom D1 fällt am Kopf an und wird zumindest teilweise in einem Wärmetauscher (10) kondensiert. Die Energie für die Auftrennung in der Destillationskolonne DKA1 (9) wird über einen Sumpfverdampfer (11) eingebracht.

Fig. 2 zeigt ebenfalls eine nicht erfindungsgemäße Verschaltung der Oligomerisierung. Zusätzlich zu der in Fig. 1 gezeigten Verfahrensführung liegt eine zusätzliche Brüdenverdichtung vor, bei der der am Kopf der Destillationskolonne DKR1 (2) anfallende Reststrom in einem Kompressor (13) verdichtet und anschließend zur Aufheizung des Reaktionsgemisches in einem Vorwärmer (14) eingesetzt wird. Bei der Rückführung vom Vorwärmer (14) erfolgt in einem weiteren Vorwärmer (12) eine Vorwärmung des Stroms vor dem Kompressor (13).

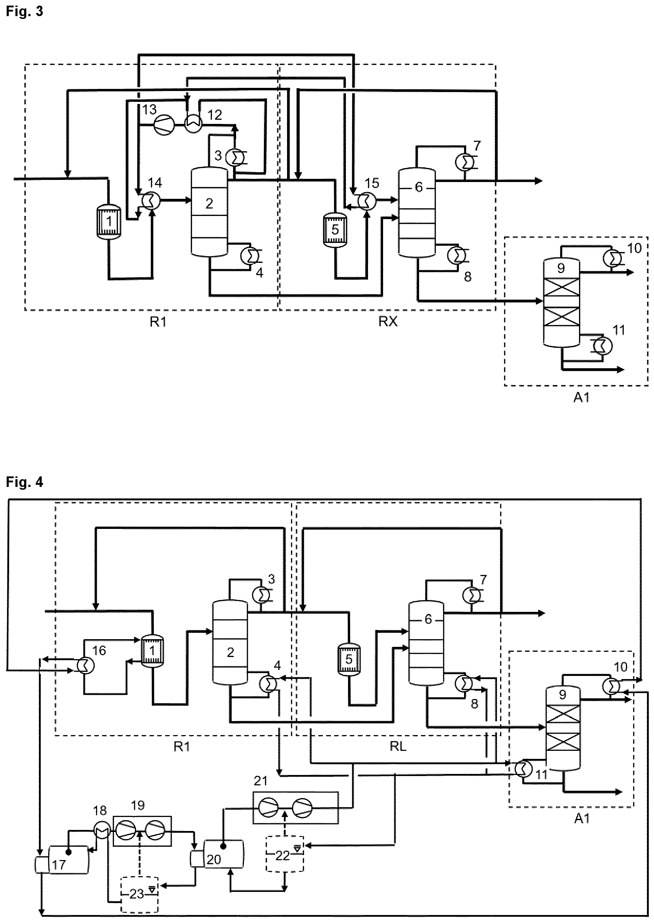

Fig. 3 zeigt ebenfalls eine nicht erfindungsgemäße Verschaltung der Oligomerisierung. Zusätzlich zu der in Fig. 2 gezeigten Verfahrensführung wird der im Kompressor (13) verdichtete Reststrom zur Aufheizung des Reaktionsgemisches der Reaktionsstufe RX in einem Vorwärmer (15) eingesetzt.

Fig. 4 zeigt eine erfindungsgemäße Verschaltung der Oligomerisierung. Zusätzlich zu der in Fig. 1 gezeigten Verfahrensführung erfolgt eine Wärmeintegration gemäß der vorliegenden Erfindung. Dabei wird die Kondensationswärme an der Destillationskolonne DKA1 (9) über den Wärmetauscher (10) und die Reaktionswärme des Reaktors (1) über den Wärmetauscher (16) mittels eines Wärmeträger A abgenommen. Mit dem dadurch erwärmten Wärmeträger A wird ein weiterer Wärmeträger B in einem Verdampfer (17) erhitzt und in einer mehrstufigen Verdichtung in einem Verdichter (19) unterzogen. Der verdichtete Wärmeträger B wird dann dazu benutzt, um einen weiteren Wärmeträger C, beispielsweise Wasser, in einem Verdampfer (20) zu erhitzen. Bei der Rückführung des Wärmeträgers B kann ein Flashbehälter (23) zwischengeschaltet werden, bei dem die dort anfallende Gasphase zwischen die Verdichterstufen des Verdichters (19) gefahren wird. Der flüssige Austrag aus dem Flashbehälter (23) kann in einem Wärmetauscher (18) zur Vorwärmung des Wärmeträgers B vor der Verdichtung eingesetzt werden. Der Wärmeträger C wird ebenfalls in einem Verdichter (21) mehrstufig verdichtet und anschließend zur Beheizung der Sumpfverdampfer (4 und 11) eingesetzt. Bei der Rückführung des Wärmeträgers C nach den Sumpfverdampfern kann ein Flashbehälter (22) zwischengeschaltet werden, bei dem die dort anfallende Gasphase zwischen die Verdichterstufen des Verdichters (21) gefahren wird. Der flüssige Austrag aus dem Flashbehälter (22) wird zum Verdampfer (20) zurückgeführt.

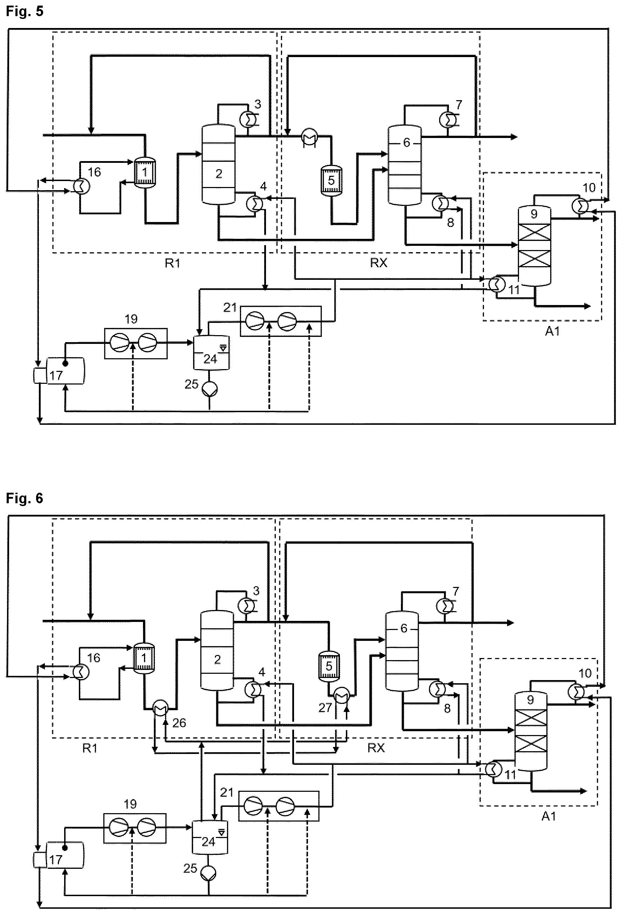

Fig. 5 zeigt ebenfalls eine erfindungsgemäße Verschaltung der Oligomerisierung. Im Unterschied zu der in Fig. 4 gezeigten Verfahrensführung gibt es nur zwei Wärmeträger. Der Wärmeträger B wird dabei im Verdampfer (17) erhitzt und anschließend einer Verdichtung im Verdichter (19), der hier vorzugsweise ein Gebläse ist, unterzogen. Der verdichtete Wärmeträger wird zu einem Flashbehälter (24) geleitet. Die dort anfallende Gasphase wird zu einem weiteren Verdichter (21) geführt, mehrstufig kompressiert und anschließend zur Beheizung der Sumpfverdampfer (4 und 11) eingesetzt. Die flüssige Phase aus dem Flashbehälter wird mittels einer Pumpe (25) zumindest teilweise zum Verdampfer (17) zurückgeführt, kann aber auch zwischen die Verdichterstufen des Verdichters (17) oder hinter den Verdichter (21) geleitet werden.

Fig. 6 zeigt ebenfalls eine erfindungsgemäße Verschaltung der Oligomerisierung. Zusätzlich zu der in Fig. 5 gezeigten Verfahrensführung wird gibt es zwei zusätzliche Vorwärmer (26 und 27). Diese Vorwärmer (26, 27) werden direkt mit der Gasphase aus dem Flashbehälter (24) als Energiequelle betrieben.

Die folgenden Beispiele sollen die Erfindung erläutern, ohne deren Anwendungsbreite, die sich aus der Beschreibung und den Patentansprüchen ergibt, einzuschränken.

### Beispiele

Alle aufgeführten Beispiele wurden mit Aspen Plus V.10 simuliert. Das kinetische Modell und die Stoffdaten wurden anhand von Betriebsdaten validiert. Alle Beispiele wurden mit den gleichen Randbedingungen simuliert. Im Feed von 40 t/h wurde ein Butenanteil von 70 wt% berücksichtigt (25 wt% 1-Buten, 30 wt% trans Buten und 15 wt% cis Buten). Der Anteil von n/i-Butan wurde mit 30 wt% berücksichtigt, wobei 3 % iso-Butan im Feed angenommen wurden. Die Feedtemperaturen zu den Reaktionsstufen wurde in allen Fällen auf 95°C konstant gehalten.

### Beispiel 1 (nicht erfindungsgemäß)

Es wurde eine Verschaltung wie sie in Fig. 1 gezeigt ist für die Simulation eingesetzt. Der Frischfeed wird mit einem Recyclatstrom von 19 t/h vermischt und mit einem Wärmeübertrager auf die Feedtemperatur von 95°C gebracht. Der Feed wird bei 25 bar abs durch eine Reaktionsstufe R1 gefahren (Rohrreaktor (1) mit Katalysator). Der Reaktor (1) wird gekühlt, um die Reaktionswärme abzuführen. Insgesamt müssen 4,2 MW abgeführt werden. Nach der ersten Reaktionsstufe wird das Reaktionsgemisch einer destillativen Trennung unterworfen. Die Destillationskolonne (2) wird bei einem Kopfdruck von 4,3 bar abs. betrieben. Die Destillationskolonne (2) hat bevorzugt eine Trennschärfe von kleiner 1 Prozent Schwersieder im Destillat, um die Reaktion nicht zu stark zu hemmen. Es entsteht eine Kopftemperatur von 45°C. Es werden 13 Böden berücksichtigt. Zur Erreichung der Reinheit wird ein Rücklauf von 16 t/h und eine Heizleistung von 5,1 MW benötigt, die durch einen 20 bar Dampf (extern zugekauft) eingebracht wird. Es entsteht eine Kondensationswärme von 5,3 MW, die durch einen Luftkühler oder einen mit Rückkühlwasser betriebenen Apparat (3) abgeführt werden.

Ein Teil des flüssigen Destillats wird zurück zur ersten Reaktionsstufe gefahren (19 t/h), der andere Teil von 20 t/h (mit einem Gewichtsanteil von n/i-Butan von 60%) zu einer weiteren, letzten Reaktionsstufe RL. Dieser Strom wird wiederum mit einem Recyclatstrom von 15 t/h vermischt und auf eine Feedtemperatur von 95°C gebracht. Es wird ein Reaktor (5) eingesetzt, der zum Reaktor (1) der ersten Stufe R1 identisch ist. Auch hier wird die Reaktionswärme über ein Prozesswasserkreislauf, der im Gleichstrombetrieben wird, abgeführt. Es müssen 1 MW abgeführt werden. Das Reaktionsgemisch gelangt hier ebenfalls zu einer destillativen Trennung. Die Destillationskolonne (6) weist insgesamt 28 Böden auf. Im Destillat der zweiten Kolonne wird ein C8-Anteil von kleiner 0,1 wt% spezifiziert. Hierfür wird ein Rücklauf von 2 t/h und eine Heizleistung von 2 MW benötigt, die wiederum durch einen externen Heizdampf eingebracht wird. Es müssen 3 MW über den Kondensator (10) abgeführt werden. Die Kolonne (9) wird ebenfalls bei 4,3 abs. betrieben. Es entsteht eine Kopftemperatur von 44°C. Der Destillatstrom wird wiederum aufgeteilt. Ein Teil von 15 t/h geht zurück zur Reaktionsstufe, der andere Teil von 14,5 t/h mit 82 wt% n/i-Butan kann zu einer weiteren Prozesseinheit oder in ein Tanklager gefahren werden. Es kann ein Umsatz von 90% erreicht werden.

Die Oligomere C8/C12/C16+ fallen im Sumpf der Destillationskolonne (6) an und werden einer weiteren Trennung in der Abtrenneinheit A1 unterzogen. In der Destillationskolonne (9) werden die C8-Isomere (bevorzugt 1-Octen und 2-Methylhepten) im Vakuum bei einem Kopfdruck von 350 mbar von den längerkettigen Oligomeren getrennt. Um eine Reinheit von bevorzugt größer 99% zu erhalten, muss ein Rücklauf von 14 t/h eingestellt werden. Es entsteht eine Kopftemperatur von 86°C. Insgesamt werden 1,4 MW Heizleistung benötigt (externer Dampf) und 3 MW Kondensationswärme müssen abgeführt werden.

Insgesamt werden dem System 11,3 MW Kondensationswärme und 5,2 MW Reaktionswärme entzogen (16,5 MW insgesamt). Dem System müssen insgesamt 8,5 MW Heizleistung zugeführt werden.

### Beispiel 2 (nicht erfindungsgemäß):

Es wurde eine Verschaltung wie sie in Fig. 2 gezeigt ist für die Simulation eingesetzt. Die Angaben aus dem Beispiel 1 gelten auch hier. Zusätzlich liegt eine Brüdenverdichtung vor, bei der die Kondensationswärme zumindest teilweise über den Verdichter (13) und die Wärmetauscher (12, 14) genutzt wird. Der Feedvorwärmer (14) trägt in diesem Beispiel 1,75 MW in das System ein. Um den Brüdenstrom auf das erforderliche Temperaturniveau zu bringen wird eine elektrische Leistung von 200 kW benötigt. Ein zu hoher Energieeintrag über den Feedverdampfer resultiert in einer Verschiebung des Temperaturprofils. Schwersieder werden nach oben gekocht. Es wird mehr Rücklauf benötigt. Die Gesamtheizleistung steigt auf 5,75 MW. Jedoch wird der externe Heizbedarf auf 4 MW an der ersten Destillationskolonne reduziert. Demzufolge wird die externe Gesamtheizleistung von 7,2 MW reduziert.

### Beispiel 3 (nicht erfindungsgemäß):

Es wurde eine Verschaltung wie sie in Fig. 3 gezeigt ist für die Simulation eingesetzt. Die Angaben aus den Beispielen 1 und 2 gelten auch hier. Zusätzlich zum Feedvorwärmer (14) wird auch der Feedvorwärmer (15) der letzten Stufe über die Brüdenverdichtung betrieben. So werden weitere 1,2 MW in das System eingetragen. Insgesamt steigt die Heizleistung der letzten Stufe auf 2,4 MW, die externe Heizleistung sinkt aber auf 1,2 MW. Hierfür muss eine elektrische Leistung über den Verdichter von 350 kW aufgebracht werden. Die externe Gesamtheizleistung beträgt in 6,4 MW.

### Beispiel 4 (erfindungsgemäß):

Es wurde eine Verschaltung wie sie in Fig. 4 gezeigt ist für die Simulation eingesetzt. Die Angaben zu den Reaktionsstufen und der Abtrennstufe aus dem Beispiel 1 gelten auch hier. Hier wird die Kondensationswärme der Vakuumdestillation und die Reaktionswärme der ersten Stufe zumindest teilweise über Wärmetauscher (10, 16) auf einen Warmwasserkreislauf übertragen. Die Wärme des Warmwasserkreislaufs wird dann eingesetzt, um einen Wärmeträger in diesem Fall Methanol bei 2,0 bar abs. im Verdampfer (17) zu verdampfen. Methanol wird einer mehrstufigen Verdichtung (19) auf 10 bar abs. unterzogen. Hier ist eine elektrische Leistung von 1,2 MW erforderlich. Durch die Kondensation des Methanols wird Wasser bei 3 bar abs. im Verdampfer (20) verdampft. Der Wasserdampf wird einer mehrstufigen Verdichtung (21) auf 20 bar abs. unterzogen. Die Verdichtung auf 20 bar abs. erfordert eine elektrische Leistung von 1,6 MW. Der Wasserdampf wird für die Beheizung der Sumpfverdampfer (4, 8, 11) benötigt. Insgesamt werden 8,5 MW externe Heizleistung durch 2,8 MW elektrische Leistung substituiert. Es wird kein externes Heizmedium (Dampf) benötigt.

### Beispiel 5 (erfindungsgemäß):

Es wurde eine Verschaltung wie sie in Fig. 5 gezeigt ist für die Simulation eingesetzt. Die Angaben zu den Reaktionsstufen und der Abtrennstufe aus dem Beispiel 1 und die weiteren Angaben aus dem Beispiel 4 gelten auch hier. Die Verschaltung ist identisch zu Beispiel 4. Hier wird Wasser an Stelle von Methanol als Wärmeträger eingesetzt. Wasser wird im leichten Vakuum (550 mbar) verdampft und auf 3 bar abs, im Verdichter (19) verdichtet. Für diesen Vorgang wird mehr elektrische Leistung benötigt. Insgesamt 3,1 MW elektrische Leistung werden benötigt um 8,5 MW externe Heizleistung (Dampf) zu subsituieren.

### Beispiel 6 (erfindungsgemäß):

Es wurde eine Verschaltung wie sie in Fig. 5 gezeigt ist für die Simulation eingesetzt. Die Angaben zu den Reaktionsstufen und der Abtrennstufe aus dem Beispiel 1 und die weiteren Angaben aus dem Beispiel 5 gelten auch hier. Es zeigt die Optimierung von Beispiel 5. Hier werden das Konzept der Feedverdampfer (26, 27) wieder aufgegriffen (vgl. Beispiele 2 und 3). Die Feedvorwärmer (26, 27) werden in diesem Beispiel mit dem erzeugten 3 bar abs. Dampf betrieben, sodass eine nicht so große Menge auf das höchste Druckniveau gebracht werden muss. Die elektrische Leistung wird auf 2,2 MW reduziert.

Eine Übersicht über die Ergebnisse der Beispiele 1 bis 6 findet sich in Tabelle 1:

**Tabelle 1: Vergleich der Energiewerte aller Beispiele**

| Beispiel | 1 | 2 | 3 | 4 | 5 | 6 |
|---|---|---|---|---|---|---|
| Heizdampf extern / MW | 8,5 | 7,2 | 6,4 | 0 | 0 | 0 |
| Elektrische Leistung / MW | 0 | 0,2 | 0,35 | 2,8 | 3,1 | 2,2 |
| Entzogene, nicht verwertete Energie / MW | 16,5 | 15 | 13,9 | 10,8 | 11,1 | 10,2 |

## Patentansprüche

1. Verfahren zur Oligomerisierung von C2- bis C8-Olefinen in mindestens zwei Reaktionsstufen R1 und RL, die jeweils mindestens einen Reaktor und mindestens eine Destillationskolonne umfassen, und einer sich an RL anschließenden Abtrennstufe A1, die mindestens eine Destillationskolonne DKA1 umfasst, wobei
ein Einsatzgemisch, welches zumindest die C2- bis C8-Olefine als Eduktolefine enthält, in dem mindestens einen Reaktor der ersten Reaktionsstufe R1 unter Verwendung eines heterogenen Katalysators einer Oligomerisierung unterworfen wird und das aus dem mindestens einen Reaktor erhaltene Reaktionsgemisch in der mindestens einen Destillationskolonne DKR1 der ersten Reaktionsstufe R1 destilliert wird, wodurch ein Reststrom RS1, der zumindest nicht umgesetzte Eduktolefine enthält, und ein Oligomerisat O1, das zumindest die gebildeten Oligomere enthält, anfallen,
RS1 zumindest teilweise zu einem Reaktor einer nachfolgenden Reaktionsstufe geführt wird;
ein Reststrom aus einer vorherigen Reaktionsstufe in dem mindestens einen Reaktor der letzten Reaktionsstufe RL unter Verwendung eines heterogenen Katalysators einer Oligomerisierung unterworfen wird und das aus dem mindestens einen Reaktor erhaltene Reaktionsgemisch in der mindestens einen Destillationskolonne DKRL der letzten Reaktionsstufe RL destilliert wird, wodurch ein Reststrom RSL, der zumindest nicht umgesetzte Eduktolefine enthält, und ein Oligomerisat OL, das zumindest die gebildeten Oligomere enthält, anfallen;
OL zumindest teilweise zur Destillationskolonne DKA1 der sich anschließenden Abtrennstufe A1 geleitet und dort destilliert wird, wodurch ein Dimerstrom D1, der zu mindestens 90 Gew.-% aus Dimeren der eingesetzten Eduktolefine besteht, und ein Sumpfstrom S1, der zumindest Trimere und höhere Oligomere der eingesetzten Eduktolefine enthält, anfallen;
der Dimerstrom D1 am Kopf der DKA1 anfällt, kondensiert wird und anschließend teilweise als Rücklauf zur DKA1 zurückgeführt und teilweise aus dem Verfahren ausgeschleust wird,
die bei der Kondensation des Dimerstroms D1 entstehende Energie und die im mindestens einen Reaktor der ersten Reaktionsstufe R1 entstehende Wärmeenergie zumindest teilweise auf einen flüssigen oder gasförmigen Wärmeträger W1 übertragen werden, wodurch ein erwärmter Wärmeträger W1.1 entsteht; und
Energie vom erwärmten Wärmeträger W1.1 auf einen weiteren flüssigen oder gasförmigen Wärmeträger W2 übertragen wird, wodurch ein erwärmter Wärmeträger W2.1 entsteht, der zunächst verdichtet und/oder erwärmt wird und über den anschließend mithilfe einer ein- oder mehrstufigen Verdichtung ein Dampfstrom erzeugt wird, mit dem zumindest die Destillationskolonnen DKA1 und DKR1 beheizt werden.

2. Verfahren nach Anspruch 1, wobei RS1 zumindest teilweise zum mindestens einen Reaktor der Reaktionsstufe R1 zurückgeführt wird.

3. Verfahren nach Anspruch 1 oder 2, wobei zwischen der Reaktionsstufe R1 und der Reaktionsstufe RL mindestens eine weitere Reaktionsstufe, die mindestens einen Reaktor und mindestens eine Destillationskolonne umfasst, vorhanden ist.

4. Verfahren nach einem der vorhergehenden Ansprüche, wobei die Reaktionsstufe R1 mindestens einen weiteren Reaktor R1.2 aufweist, der zum Reaktor R1.1 parallel oder in Reihe geschaltet wird, vorzugsweise in Reihe geschaltet vorliegt.

5. Verfahren nach Anspruch 4, wobei auch die am Reaktor 2.1 entstehende Energie zumindest teilweise auf den Wärmeträger W1 übertragen wird.

6. Verfahren nach einem der vorhergehenden Ansprüche, wobei das Verfahren in mindestens einer weiteren Reaktionsstufe R2 durchgeführt wird, wobei Reaktionsstufe R2 mindestens einen Reaktor R2.1 und mindestens eine Destillationskolonne DKR2 umfasst.

7. Verfahren nach einem der vorhergehenden Ansprüche, wobei der erzeugte Dampfstrom auch zur Beheizung der Destillationskolonne DKR2 eingesetzt wird.

8. Verfahren nach einem der vorherigen Ansprüche, wobei der Wärmeträger W1 aus der Gruppe, bestehend aus Wasser; Alkoholen, Alkohol-Wasser-Lösungen; Salz-Wasser-Lösungen; Mineralölen, wie zum Beispiel Dieselöle; Thermalölen, wie zum Beispiel Silikonöle; biologische Ölen, wie zum Beispiel Limonen; und aromatischen Kohlenwasserstoffen, wie zum Beispiel Dibenzyltoluol, ausgewählt wird.

9. Verfahren nach einem der vorherigen Ansprüche, wobei der Wärmeträger W2 aus der Gruppe, bestehend aus Wasser; Alkoholen, Alkohol-Wasser-Lösungen; Salz-Wasser-Lösungen; Mineralölen, wie zum Beispiel Dieselöle; Thermalölen, wie zum Beispiel Silikonöle; biologische Ölen, wie zum Beispiel Limonen; und aromatischen Kohlenwasserstoffen, wie zum Beispiel Dibenzyltoluol, ausgewählt wird.

10. Verfahren nach einem der vorhergehenden Ansprüche, wobei der erwärmte Wärmeträger W2.1 verdichtet wird und als verdichteter Wärmeträger W2.2 anfällt.

11. Verfahren nach Anspruch 10, wobei Energie vom verdichteten Wärmeträger W2.2 auf einen weiteren Wärmeträger W3 übertragen wird, wodurch ein erwärmter Wärmeträger W3.1 entsteht, der ein- oder mehrstufig, vorzugsweise mehrstufig, verdichtet wird und anschließend als Dampfstrom zur Beheizung aller in den Reaktionsstufen und aller in der Abtrennstufe vorhandenen Destillationskolonnen eingesetzt wird.

12. Verfahren nach Anspruch 11, wobei der Wärmeträger W3.1 durch die ein- oder mehrstufige Verdichtung auf einen Druck im Bereich von 5 bis 40 bar, vorzugsweise 10 bis 30 bar, vorzugsweise 12 bis 25 bar gebracht wird.

13. Verfahren nach einem der vorherigen Ansprüche, wobei das Reaktionsgemisch vorgewärmt wird, bevor es ein die Destillationskolonne DKR1 eingeleitet wird.

14. Verfahren zur Oligomerisierung nach einem der vorhergehenden Ansprüche, wobei in den Reaktoren der einzelnen Reaktionsstufen ein Oligomerisierungskatalysator eingesetzt wird, welcher eine Nickelverbindung auf einem Alumosilicat-Trägermaterial umfasst und welcher vorzugsweise weniger als 0,5 Gew.-% Titandioxid und Zirkoniumdioxid in seiner Gesamtzusammensetzung enthält.

15. Verfahren zur Oligomerisierung nach einem der vorhergehenden Ansprüche, wobei das Verfahren ein Verfahren zur Oligomerisierung von C3- bis C6-Olefinen, vorzugsweise von C3- bis C5-Olefinen, besonders bevorzugt von C4-Olefinen ist.
